Europäisches Patentamt

(19) European Patent Office

Office européen des brevets

(11) Publication number : **0 396 317 B1**

(12) **EUROPEAN PATENT SPECIFICATION**

(45) Date of publication of patent specification :
01.09.93 Bulletin 93/35

(21) Application number : 90304465.9

(22) Date of filing : 25.04.90

(51) Int. Cl.[5] : **A61K 31/225,** A61K 31/19,
A61K 7/22, A61K 7/16,
// (A61K31/225, 31:19,
31:155)

(54) **Pharmaceutical composition.**

The file contains technical information
submitted after the application was filed and
not included in this specification

(30) Priority : 29.04.89 GB 8909906

(43) Date of publication of application :
07.11.90 Bulletin 90/45

(45) Publication of the grant of the patent :
01.09.93 Bulletin 93/35

(84) Designated Contracting States :
BE CH DE ES FR GB LI

(56) References cited :
EP-A- 0 110 568
GB-A- 843 133
GB-A- 848 066
GB-A- 2 092 442

(73) Proprietor : BIOREX LABORATORIES LIMITED
2 Crossfield Chambers, Gladbeck Way
Enfield, Middlesex EN2 7HT (GB)

(72) Inventor : Baxendale, Lily
40 Galley Lane
Arkley, Barnet, Herfordshire EN5 4AJ (GB)

(74) Representative : Williams, John Francis et al
WILLIAMS, POWELL & ASSOCIATES 34
Tavistock Street
London WC2E 7PB (GB)

EP 0 396 317 B1

## Description

The present invention is concerned with new pharmaceutical compositions for treating diseases of mucous membranes which are externally accessible, especially of the oral cavity and of the vaginal and anal regions.

Caries, gingivitis, periodontitis and recurrent aphthous ulceration are the most common diseases in the oral cavity. Furthermore, mouth ulcers frequently occur in neutropenic patients and in patients undergoing chemotherapy and/or radiation therapy.

Aphthous ulceration principally occurs in the late teens or early twenties and affects 20% of the population of both sexes, with a slight predominance in females. In the minor form, ulcers heal in less than two weeks, whilst in the major form healing takes several weeks or months. The aetiology is obscure but an autoimmune background is often suspected. A cross-reaction between oral mucosal antigens and oral Streptococci has been described as taking place in recurrent aphthous ulcer patients. The ulcers may not be infective in origin but secondary infection exacerbates the severity and the duration.

One of the oldest methods of treatment for recurrent aphthous ulceration is steroids. In most patients with aphthous ulcer, the steroids are either ineffective or are contraindicated, especially when virus infection is suspected as a cause or consequence of the ulceration. Antiseptic mouthwashes and antibiotics have been used for the treatment of the aphthous ulceration. It is believed that the reduction in the severity and the duration of the aphthous ulcer is due to the prevention of the secondary infection rather than a healing effect.

Carbenoxolone (glycyrrhetinic acid hydrogen succinate; see GB-A-843,133), which has a long clinical history, and many other derivatives of glycyrrhetinic acid, have been shown to promote healing of gastric and duodenal ulcers. Most probably, their mode of action relies on cytoprotection, accelerating the maturation of gastric and intestinal epithelial cells and stimulating mucous secretion. GB-A-848,066 describes compositions for dental use which can contain glycyrrhetinic acid and/or derivatives thereof as disclosed in GB-A-843,133. Many glycyrrhetinic acid derivatives also have anti-inflammatory properties which are not mediated directly via an inhibition of prostaglandin synthesis like most other anti-inflammatory drugs.

For the above activities, glycyrrhetinic acid derivatives, including carbenoxolone, have been especially formulated in a gel form with a muco-adhesive property and have been used for several years for the treatment of aphthous ulceration (see GB-A-1,124,976). These derivatives have also been prepared in the form of mouthwash granules (see GB-A-2,092,442) and successfully used not only for minor and major aphthous ulceration but also for the treatment of oral lichen planus in both erosive and symptomatic non-erosive forms.

Furthermore, EP-A-0 110 568 discloses oral compositions containing an antimicrobial, such as chlorhexidine, but makes no mention of the problems associated with the use of chlorhexidine or of any possibilities of overcoming such problems.

Clinical studies have demonstrated the superiority of a carbenoxolone mouthwash over other traditional treatments for aphthous ulceration. It has been used successfully for the treatment of most if not all types of mouth ulceration.

In another study in vitro, it has been found that carbenoxolone mouthwash granules have no demineralising action on teeth.

Chlorhexidine is widely accepted and well documented for the inhibition of plaque formation and prevention of gingivitis and periodontitis. Chlorhexidine mouth rinse is used in a 0.2% concentration but a 0.12% concentration has been found to be as effective as the higher concentrations.

Numerous clinical studies have been published using chlorhexidine mouth rinse, gum and other forms as a preventative and therapeutic agent for various clinical applications for dental and oral indications. Chlorhexidine mouth rinse and tooth gel are commercially available but its bitter taste and staining of tooth and soft tissue have limited the use due to poor patient compliance.

Whereas carbenoxolone is an anionic compound, chlorhexidine is a cationic compound and, as such, it is compatible with other cationic and non-ionic compounds but not with anionic compounds, such as carbenoxolone. This would present difficulties in combinations because of its chemical nature. Immediate precipitation of a carbenoxolone/chlorhexidine complex in a colloidal form is seen in a wide range of pH values. At a low pH (less than 3.5), carbenoxolone can be separated from the suspension by filtration. At a pH of 3.5 or less, chlorhexidine will remain in solution. Chlorhexidine, on the other hand, can be separated by filtration at pH 9.0 or more, leaving carbenoxolone in solution. When the colloidal particles are separated by filtration at neutral pH (pH 7.0 ± 0.5), washed and dried under vacuum, the complex is insoluble in propylene glycol, polyethylene glycol, acetone and ethanol but soluble in methanol. When the methanolic solution of the complex is run on T.L.C., the complex cleaves into two moieties. One moiety runs parallel to chlorhexidine, while the second runs parallel to carbenoxolone. This indicates that this complex is not a single salt but a reversible combination which can be split to release the two separate therapeutic activities.

The alcohol-induced ulcer test in rats showed that carbenoxolone in the combination form is equally as

2

effective as carbenoxolone alone. Similarly, tests carried out on combination mouthwash granules showed that the antimicrobial activity of chlorhexidine is reduced but it is still effective against both gram negative and gram positive micro-organisms. Further tests on a wider range of micro-organisms have been carried out using ten different micro-organisms from which it was concluded that all the test organisms were inhibited in the 10 - 500 μg range of chlorhexidine and that any product containing 1% would still be effective even if there is a 20-fold reduction in activity.

In the combination form, both chlorhexidine disadvantages are either completely eliminated or significantly reduced, testing for staining having been carried out in vitro on excised teeth.

Preliminary results from clinical studies suggest that carbenoxolone in a combination form with chlorhexidine is much more effective than carbenoxolone mouthwash granules or chlorhexidine alone.

Thus, according to the present invention, there is provided a pharmaceutical composition for treating diseases of mucous membranes, comprising chlorhexidine in the form of a salt with a non-toxic, pharmaceutically acceptable inorganic or organic acid and at least one derivative of glycyrrhetinic acid (as hereinafter defined).

The compositions according to the present invention can contain 1 to 10 and preferably 1 to 2.5 parts by weight of the glycyrrhetinic acid derivative per 1 part by weight of chlorhexidine salt, an especially preferred composition containing equal parts by weight of both components.

The compositions according to the present invention can have a pH of from about 7 to about 9 and preferably of from 7.3 to 8.2.

Since the compositions according to the present invention are intended to be used for the treatment of diseases of the oral cavity, they are preferably in the form of solid mixtures, oral gels, aqueous solutions or suspensions or toothpastes.

The glycyrrhetinic acid derivatives used according to the present invention are the 3-0-acyl derivatives and especially those in which the acyl radical contains a carboxyl group (see GB-A-843,133 and GB-A-1,387,499), the esters of glycyrrhetinic acid and of 3-acyl derivatives of glycyrrhetinic acid (see GB-A-1,255,672) and also the 2 (ω-carboxyalkanoyl alkanoyl (and cycloalkanoyl)-oxymethyl)-glycyrrhetinic acid derivatives (see GB-A-1,476,053). In those cases in which the glycyrrhetinic acid derivative contains one or more free carboxyl groups, such groups are preferably salified and used, for example, in the form of alkali metal salts, the sodium salts being especially preferred. Of the large number of glycyrrhetinic acid derivatives which can be used according to the present invention, the preferred ones include the disodium salt of glycyrrhetinic acid hydrogen succinate (carbenoxolone sodium), the disodium salt of mono-(glycyrrhet-3-yl)-cis-cyclohexane-1,2-dicarboxylic acid and cinnamyl glycyrrhetate.

Since chlorhexidine is strongly alkaline, according to the present invention it is used in the form of a salt with a non-toxic, pharmaceutically acceptable inorganic or organic acid. Appropriate acids for this purpose include hydrochloric acid, hydrofluoric acid, sulphuric acid, phosphoric acid, difluorophosphoric acid, acetic acid, gluconic acid, propionic acid, malonic acid, malic acid, tartaric acid, succinic acid and lactic acid.

According to a preferred embodiment of the present invention, the compositions can also contain at least one non-toxic oxygen-generating compound, for example a perborate or percarbonate. Furthermore, an appropriate per acid can also be used for salifying the chlorhexidine.

The compositions according to the present invention can, if desired, also contain a fluorine compound of the kind which is conventionally used in toothpastes, mouth washes and the like, for example sodium monofluorophosphate.

When a composition according to the present invention is used as a mouthwash, we have found that 30 to 50 ml. is an adequate volume for a single mouthwash and that about 2 g. of the composition is sufficient to provide the desired effect. Consequently, for ease of use, a composition according to the present invention intended for use as a mouthwash is preferably packed into individual sachets, each of which contains 2 g. of the composition, a plurality of such sachets being packed into a larger container in order to provide an adequate course of treatment for a patient.

The following Examples are given for the purpose of illustrating the present invention, the percentages being by weight unless otherwise stated:

Example 1.

Mouth rinse

Preliminary experiment suggests that, in aqueous preparation, carbenoxolone in a complex form with chlorhexidine is more stable, with or without sodium carboxymethylcellulose and glycerol, than carbenoxolone in aqueous solution. Physically, at ambient temperature and 30°C. storage conditions for at least 6 months, the colloidal form of the combination is stable. With minimum shaking, the settled colloidal particles are uniformly distributed.

Formulation I

| carbenoxolone sodium | | 0.2% |
| chlorhexidine gluconate | | 0.2% |
| purified water | add to | 100 ml. |

Method of preparation

1. Dissolve the carbenoxolone sodium in 40% of the purified water.
2. Dilute the chlorhexidine gluconate solution with 40% of the purified water.
3. At a high speed of stirring, gradually add the diluted chlorhexidine solution to the carbenoxolone solution.
4. Complete the volume with purified water.

Formulation II

| carbenoxolone sodium | | 0.2% |
| chlorhexidine gluconate | | 0.2% |
| sodium carboxymethylcellulose | | 0.4% |
| glycerol | | 10.0% |
| purified water | add to | 100 ml. |

Method of preparation

1. Suspend the sodium carboxymethylcellulose in the glycerol.
2. Gradually add the suspension to 60% of the purified water, with stirring.
3. Dissolve the carbenoxolone sodium in the mixture of Step 2, with stirring.
4. Dilute the chlorhexidine gluconate with 40% of the purified water.
5. Gradually add the diluted chlorhexidine gluconate solution to the carbenoxolone solution with high speed stirring.

No preservatives are added to the above two formulations because chlorhexidine has an antimicrobial activity. Sweetening agents (such as saccharin), flavouring agents (such as peppermint oil, spearmint oil, cardamom oil or cinnamon oil) and colouring agents (such as an appropriate chlorophyllin) can be added, if desired, to the above two formulations.

Example 2.

Mouthwash granules

Formulation:

| carbenoxolone sodium | 1% |
| chlorhexidine gluconate | 1% |
| disodium edetate | 0.5% |
| sodium citrate (comminuted) | 20.0% |
| lactose | 77.1% |
| peppermint oil | 0.4% |

Method of granulation

1. Tumble mix the carbenoxolone sodium and disodium edetate with an equal amount of lactose and transfer to a bowl mixer.
2. Add the comminuted sodium citrate and half of the lactose and mix at a low speed for 5 minutes.
3. Add the remaining lactose and further mix for 5 minutes.
4. Dilute the chlorhexidine gluconate solution with an equal amount of distilled water.
5. Set the mixer at low speed and gradually add the diluted chlorhexidine gluconate solution until uniform moist mass is achieved.

6. Pass the moist mass through a 0.8 mm (No.20) sieve and transfer to the vacuum dryer adjusted to 60°C.

7. Pass the dried granules through a 1 mm (No. 16) sieve and weigh.

8. Weigh the required amount of peppermint oil (equivalent to 0.4% of the dried granules) and add gradually at a low mixing speed.

No preservatives and colouring agents are required. Various flavours, such as spearmint, cardamon, cinnamon and clove oil can be used, if desired. The pH of 2 g. of granules dissolved in 40 ml. of distilled water is from 6.8 to 7.0.

Example 3.

Tooth gel

Formulation:

```
carbenoxolone sodium                        0.2%

chlorhexidine gluconate                     0.2%
                      ®
Carbopol  940                               1.0%

sodium hydroxide, 10% aqueous
                    solution              Q.S.

titanium dioxide                          0.5%

distilled water                add to  100 g.
```

Method of preparation for 400 g. batch.

1. Boil and cool to 40 - 50°C. an excess of distilled water. Transfer 270 g. of the required distilled water into a 500 ml. beaker.

2. Place the stirrer as deeply as possible into the beaker and stir at low speed.

3. Gradually sprinkle the Carbopol® 940 on to the surface of the water. Increase the speed of stirring when necessary (as the viscosity increases) until the Carbopol® 940 completely dissolves.

4. Adjust the pH to 7 with 10% aqueous sodium hydroxide solution.

5. Dilute the chlorhexidine gluconate solution (4.0 ml.) to 40 ml. with distilled water.

6. Dissolve the carbenoxolone sodium in 40 ml. of distilled water and mix, with high speed of stirring, with the diluted chlorhexidine gluconate solution.

7. Add gradually to the gel with minimum stirring in order to avoid air entrapment.

8. Check the pH end adjust with 4% aqueous sodium hydroxide solution, if necessary.

9. Suspend the titanium dioxide (2.0 g.) in 5 ml. of distilled water and add to the gel, with minimum stirring.

10. Adjust the weight to 400 g. with distilled water.

The product of the above formulation is a brilliant white gel with a high consistency.

If desired, sweetening agents (such as sodium saccharin), flavouring agents (such as peppermint, spearmint, cardamom, cinnamon or clove oil) and colouring agents (such as an appropriate chlorophyllin) may be added. An anti-caries additive (such as sodium monofluorophosphate) may also be used, especially for use by children.

Example 4.

Tooth paste

Formulation:

| | |
|---|---|
| alumina trihydrate (tooth paste grade) | 50.0% |
| carbenoxolone sodium | 1.0% |
| chlorhexidine gluconate solution | 5% |
| sodium carboxymethylcellulose, high viscosity grade and low viscosity grade | 1.1% |
| glycerol | 17.5% |
| sodium monofluorophosphate | 0.8% |
| sodium saccharin | 0.2% |
| peppermint oil | 0.2% |
| spearmint oil | 0.2% |
| water | 23.8% |

Method of preparation of a 500 g. batch

Method I

1. Mix the glycerol with distilled water (less 10 ml.) in a 500 ml. beaker.
2. Sprinkle the sodium carboxymethylcellulose (0 : 1.1 or 0.5 : 0.6 of low viscosity grade : high viscosity grade) on to the glycerol-distilled water mixture with a minimum of stirring (increase when necessary) until completely dissolved.
3. Add the sodium saccharin, sodium monofluorophosphate and carbenoxolone sodium with continuous stirring until dissolved.
4. Add the chlorhexidine gluconate dropwise, with stirring.
5. Gradually add the alumina trihydrate with continuous stirring.
6. Disperse the titanium dioxide in the remaining 10 ml. of distilled water and add to the mixture, with continuous stirring, until a homogeneous paste is produced.
7. Add the flavouring agents with minimum stirring.

Method II

1. Disperse the sodium carboxymethylcellulose in the glycerol without stirring to avoid air entrapment.
2. Dissolve the sodium saccharin, sodium monofluorophosphate and carbenoxolone sodium in water.
3. Gradually add the chlorhexidine gluconate solution dropwise with high speed stirring and leave to stand until all air bubbles have escaped.
4. Add the titanium dioxide with minimum stirring.
5. Gradually add the alumina trihydrate, with stirring, until a homogeneous product is obtained and deaerate under vacuum, if necessary.
6. Add the glycerol phase to the aqueous phase with minimum stirring to avoid air entrapment.

Special ultra-fine alumina trihydrate grade with fruit flavours, such as strawberry, banana or apricot, and matching colours can be used for children's tooth paste.

A clinical trial has been carried out in order to demonstrate the efficacy of pharmaceutical compositions according to the present invention.

The composition used had the following formulation:

| | |
|---|---|
| carbenoxolone sodium BP | 20 mg. |
| chlorhexidine gluconate | 20 mg. |
| polyethylene glycol 4000 | 250 mg. |
| polyvinylpyrrolidone | 50 mg. |
| citric acid | 22 mg. |
| sucrose | 819 mg. |
| lactose | 819 mg. |
| | 2000 mg. |

For use, the above composition was dissolved in 30 to 50 ml. of water.

Ten patients with haematological malignancies and undergoing intensive chemotherapy/radiation therapy were investigated during 10 treatment schedules in a non-randomised study to assess the efficacy of the mouthwash composition according to the present invention in the prevention of mucosal ulceration.

The use of the mouthwash was commenced 24 hours prior to the treatment and continued until the neutrophil counts rose to $0.5 \times 10^9$/litre. Mouth swabs were examined for pathogens and viral antibody titres were estimated at regular intervals. The mucosal changes were assessed daily by a scoring system.

The group comprised 5 patients treated for acute myeloid leukaemia, 3 for acute lymphoblastic leukaemia and 2 for non-Hodgkin's lymphoma. The mean period of neutropenia was 14.5 days.

In 7 patients, the mucosal changes were mild without ulceration. Two patients developed ulcers on the tongue and only one had severe symptoms due to generalised mucositis. No viruses were isolated from any of the patients and there was no rise in viral antibody titres. 8 patients became pyrexial with positive blood cultures (gram positive cocci) in 5. The mouthwash according to the present invention was well tolerated and no side effects were observed.

These results demonstrate the usefulness of the compositions according to the invention for the prevention of mucosal ulceration in patients with prolonged neutropenia.

## Claims

1. A pharmaceutical composition for treating diseases of mucous membranes, comprising chlorhexidine in the form of a salt with a non-toxic, pharmaceutically acceptable inorganic or organic acid and at least one glycyrrhetinic acid derivative selected from the 3-0-acyl derivatives thereof, the acyl radical of which can contain a carboxyl group, esters of glycyrrhetinic acid and of 3-acyl derivatives of glycyrrhetinic acid and 2-($\omega$-carboxyalkanoyl- and -cycloalkanoyl-oxymethyl)-glycyrrhetinic acid derivatives, as well as the salts of those glycyrrhetinic acid derivatives which contain one or more free carboxyl groups.

2. A pharmaceutical composition according to claim 1, wherein it contains 1 to 10 parts by weight of glycyrrhetinic acid derivative per 1 part by weight of chlorhexidine salt.

3. A pharmaceutical composition according to claim 2, wherein it contains 1 to 2.5 parts by weight of glycyrrhetinic acid derivative per 1 part by weight of chlorhexidine salt.

4. A pharmaceutical composition according to claim 3, wherein it contains equal parts by weight of glycyrrhetinic acid derivative and chlorhexidine salt.

5. A pharmaceutical composition according to any of the preceding claims, wherein it additionally contains at least one non-toxic, oxygen-generating compound.

6. A pharmaceutical composition according to claim 5, wherein the oxygen-generating compound is a perborate or a percarbonate.

7. A pharmaceutical composition according to any of the preceding claims, wherein it contains a fluorine compound suitable for oral use.

8. A pharmaceutical composition according to claim 7, wherein the fluorine compound is sodium monofluorophosphate.

9. A pharmaceutical composition according to any of the preceding claims, wherein the glycyrrhetinic acid derivative is the disodium salt of glycyrrhetinic acid hydrogen succinate, the disodium salt of mono(glycyrrhet-3-yl)-cis-cyclohexane-1,2-dicarboxylic acid or cinnamyl glycyrrhetate.

10. A pharmaceutical composition according to any of the preceding claims which is in the form of a solid mixture, an oral gel, an aqueous solution or suspension or a toothpaste.


**Patentansprüche**

1. Eine pharmazeutische Zusammensetzung zur Behandlung von Schleimhauterkrankungen, enthaltend Chlorhexidin in Form eines Salzes mit einer ungiftigen, pharmazeutisch akzeptablen anorganischen oder organischen Säure und mindestens einen Glycyrrhetinsäure-Abkömmling, der ausgewählt ist aus deren 3-0-acyl Abkömmlingen, deren Acylrest eine Carboxylgruppe enthalten kann, ferner Ester aus Glycyrrhetinsäure und 3-Acyl-Abkömmlingen der Glycyrrhetinsäure und 2-$\omega$-Carboxyalkanoyl- und cyclo-alkanoyl-oxymethyl)-Glycyrrhetinsäure-Abkömmlinge, sowie die Salze derjenigen Glycyrrhetinsäure-Abkömmlinge, die ein oder mehrere freie Carboxylgruppen enthalten.

2. Eine pharmazeutische Zusammensetzung nach Anspruch 1, die 1 bis 10 Gewichtsanteile des Glycyrrhetinsäure-Abkömmlings pro 1 Gewichtsanteil von Chlorhexidinsalz enthält.

3. Eine pharmazeutische Zusammensetzung nach Anspruch 2, die 1 bis 2,5 Gewichtsanteile eines Glycyrrhetinsäure-Abkömmlings pro 1 Gewichtsanteil Chlorhexidinsalz enthält.

4. Eine pharmazeutische Zusammensetzung nach Anspruch 3, die gleiche Gewichtsanteile eines Glycyrrhetinsäure-Abkömmlings und von Chlorhexidinsalz enthält.

5. Eine pharmazeutische Zusammensetzung gemäß einem der vorhergehenden Ansprüche, die zusätzlich mindestens eine ungiftige Sauerstoff erzeugende Verbindung enthält.

6. Eine pharmazeutische Zusammensetzung nach Anspruch 5, bei der die Sauerstoff erzeugende Verbindung ein Perborat oder ein Percarbonat ist.

7. Eine pharmazeutische Zusammensetzung nach einem der vorhergehenden Ansprüche, dadurch gekennzeichnet, daß sie eine fluorhaltige Verbindung, die für die Verwendung im Mund geeignet ist, enthält.

8. Eine pharmazeutische Zusammensetzung nach Anspruch 7, bei der die fluorhaltige Verbindung Natriummonofluorphosphat ist.

9. Eine pharmazeutische Zusammensetzung nach einem der vorhergehenden Ansprüche, bei der der Glycyrrhetinsäure-Abkömmling das Dinatriumsalz des Glycyrrhetinsäure-hydrogen-succinats, das Dinatriumsalz der Mono-(Glycyrrhet-3-yl)-cis-cyclohexan-1-,2-dicarboxylsäure oder Cinnamyl Glycyrrhetat ist.

10. Eine pharmazeutische Zusammensetzung nach einem der vorhergehenden Ansprüche, die in Form einer festen Mischung, eines Mundgels, einer wässrigen Lösung oder Suspension oder in Form einer Zahnpasta vorliegt.

**Revendications**

1. Composition pharmaceutique permettant de traiter des affections des muqueuses, comprenant de la chlorhexidine sous la forme d'un sel avec un acide minéral ou organique non-toxique pharmaceutiquement acceptable et au moins un dérivé de l'acide glycyrrhétinique choisi parmi les dérivés de 3-0-acyle et en particulier ceux dans lequel le radical acyle contient un groupe carboxyle, les esters de l'acide glycyrrhétinique et de dérivés de 3-acyle de l'acide glycyrrhétinique et également les dérivés de l'acide 2-(omêga-carboxy-alkanoyl - et cycloalkanoyl)-oxyméthyl)-glycyrrhétinique, ainsi que les sels des dérivés de l'acide glycyrrhétinique qui contiennent un ou plusieurs groupes carboxyle libres.

2. Composition pharmaceutique selon la revendication 1, contenant 1 à 10 parties en poids de dérivé d'acide glycyrrhétinique par partie en poids de sel de chlorhexidine.

3. Composition pharmaceutique selon la revendication 2, contenant 1 à 2,5 parties en poids de dérivé d'acide glycyrrhétinique par partie en poids de sel de chlorhexidine.

4. Composition pharmaceutique selon la revendication 3, contenant des parties en poids égales de dérivé d'acide glycyrrhétinique et de sel de chlorhexidine.

5. Composition pharmaceutique selon l'une quelconque des revendication précédentes, contenant en outre au moins un composé générateur d'oxygène non toxique.

6. Composition pharmaceutique selon la revendication 5, dans laquelle le composé générateur d'oxygène est un perborate ou un percarbonate.

7. Composition pharmaceutique selon l'une quelconque des revendications précédentes, contenant un composé fluoré convenant à un usage oral.

8. Composition pharmaceutique selon la revendication 7, dans laquelle le composé fluoré est le monofluorophosphate de sodium.

9. Composition pharmaceutique selon l'une quelconque des revendications précédentes, dans laquelle le dérivé de l'acide glycyrrhétinique est le sel disodique du succinate acide de l'acide glycyrrhétinique, le sel disodique de l'acide mono-(glycyrrhat-3-yl)-cis-cyclohexane-1,2-dicarboxylique ou le glycyrrhétate de cynnamyle.

10. Composition pharmaceutique selon l'une quelconque des revendications précédentes, se présentant sous la forme d'un mélange solide, d'un gel oral, d'une solution ou d'une suspension aqueuse ou encore d'une pâte dentifrice.